Europäisches Patentamt

European Patent Office

Office européen des brevets

(11) Veröffentlichungsnummer: **0 217 970**
**A1**

# EUROPÄISCHE PATENTANMELDUNG

(21) Anmeldenummer: 85112339.8

(22) Anmeldetag: 28.09.85

(51) Int. Cl.⁴: **A61B 5/10** , A61H 1/00 , A63B 21/00

(43) Veröffentlichungstag der Anmeldung:
15.04.87 Patentblatt 87/16

(84) Benannte Vertragsstaaten:
AT BE CH DE FR GB IT LI LU NL SE

(71) Anmelder: GGT Medizin-Electronic-Systeme GmbH
Badstrasse 7
D-8000 München 70(DE)

(72) Erfinder: Ott, Emil, Dipl.-Ing.
Wiebekingstrasse 2a
D-8000 München 50(DE)
Erfinder: König, Herbert, Prof. Dr.-Ing.
Simmernstrasse 5
D-8000 München 40(DE)

(74) Vertreter: Hansmann, Dierk, Dipl.-Ing.
Jessenstrasse 4
D-2000 Hamburg 50(DE)

(54) **Vorrichtung zur Ermittlung und Verbesserung der Funktionsfähigkeit des Bewegungsapparates des Menschen.**

(57) Eine Vorrichtung zur Ermittlung der Funktionsfähigkeit des Bewegungsapparates des Menschen nach objektiven Kriterien und die quantitative Erfassung des Grades der Funktionsfähigkeit ist bekannt, sie weist jedoch einen sehr komplexen Aufbau auf, und zudem nicht zur Abgabe der eigentlichen erforderlichen Leistung befähigt ist und andererseits verhältnismäßig geräuschvoll und in einigen Betriebszuständen diskontinuierlich arbeitet. Die Erfindung schlägt eine Vorrichtung vor, die bei der gewünschten großen Leistungsabgabe einen verhältnismäßig einfachen Aufbau hat und leicht steuerbar ist. Dazu wird die Extremität in eine Haltevorrichtung gelegt, die durch ein Hydraulikantriebsaggregat (150) über ein hydraulisch angetriebenes Kolben-Zylinder-Antriebssystem (151) derart angetrieben wird, daß die Haltevorrichtung (12) in Abhängigkeit der Steuerung des Kolben-Zylinder-Antriebssystems (151) um ihre Achse (15) schwenkbar ist. Die von der Haltevorrichtung (12) aufge nommene Gelenkachse einer Extremität wird dabei entsprechend der Achse (14) der Haltevorrichtung (12) gedreht.

Fig. 2

## Vorrichtung zur Ermittlung und Verbesserung der Funktionsfähigkeit des Bewegungsapparates des Menschen

Die Erfindung betrifft eine Vorrichtung zur Ermittlung und Verbesserung der Funktionsfähigkeit des Bewegungsapparates des Menschen, insbesondere seiner Extremitäten, mit einer Aufnahme der extremitätdienenden Haltevorrichtung, die durch einen Antriebsmotor um eine der in der Haltevorrichtung aufgenommenen Gelenkachse einer Extremität entsprechenden Achse drehbar ist.

Eine Vorrichtung dieser Art ist bekannt - (Europäische Patentanmeldung Nr. 84116217.5-), mit der es möglich ist, die Funktionsfähigkeit des Bewegungsapparates des Menschen nach objektiven Kriterien zu ermitteln und den Grad der Funktionsfähigkeit quantitativ sowohl beim kranken Menschen als auch beim gesunden Menschen zu erfassen. Darüber hinaus ist mit der bekannten Vorrichtung eine Verbesserung der Funktionsfähigkeit eileq als defekt erkannten Bewegungsapparates unter Festlegung quantitativer Bewegungsparameter ebenfalls möglich, so daß ein physikalisches Training nicht mehr durch ausgebildete Therapeuten von Hand durchgeführt werden muß, sondern diese Therapie vielmehr durch die bekannte Vorrichtung beliebig oft und nach Art und Größe reproduzierbaren Bewegungskriterien ausgeführt werden kann.

Bei der bekannten Vorrichtung wird die Haltevorrichtung, in der die Extremität des Menschen, beispielsweise Arm oder Bein aufgenommen wird, durch einen elektronisch gesteuerten Schrittmotor über Kupplungs-und Getriebeeinrichtungen angetrieben, wobei jeweils ein kompletter Antriebssatz zum Antrieb je einer Haltevorrichtung verwendet wird. Abgesehen von dem erheblichen Aufwand von derartigen Antriebsmitteln, die beispielsweise bei 3 in einer Vorrichtung vorgesehenen Haltevorrichtungen dreifach vorgesehen werden müssen, stellen die dann erforderlichen Schrittmotoren zum Antrieb der Haltevorrichtung eine dreifache Fehlerquelle dar. Darüber hinaus können Schrittmotoren nur mit erheblichem elektronischem Aufwand gesteuert werden, so daß auch die elektronischen Einrichtungen wiederum nur mit erheblichem Aufwand hergestellt werden können und zudem eine, bedingt durch den erforderlichen Schaltungsaufwand, Fehlerquelle darstellen.

Schließlich ist die abgegebene Leistung von Schrittmotoren physikalisch bedingt begrenzt, was sich insbesondere bei vielfach erforderlichen hohen Beschleunigungen der Haltevorrichtung als Mangel erweist. Darüber hinaus weist ein Schrittmotor die Eigenschaft auf, daß er bei einer Last, die größer als das maximal verfügbare Drehmoment bei einer bestimmten Drehgeschwindigkeit der Haltevorrichtung ist, zum Stillstand kommt, was sich ebenfalls als Mangel erweist.

Es ist deshalb Aufgabe der Erfindung, eine Vorrichtung zu schaffen, die gegenüber der bekannten Vorrichtung erheblich einfacher im Aufbau ist, die darüber hinaus eine erheblich größere Leistung abzugeben vermag, die ein erheblich geräuschärmeres und gleichmäßigeres Betriebs-verhalten zeigt und die Haltevorrichtung erheblich stärker als bisher beschleunigen kann, so daß die Ermittlung der Funktionsfähigkeit des Bewegungsapparates des Menschen für diagnostische und Testzwecke sowohl kranker als auch gesunder Menschen anhand objektiver und qualitativer Bewegungsparameter ebenso wie deren Einsatz für therapeutische Zwecke nochmals verbessert wird.

Gelöst wird die Aufgabe gemäß der Erfindung dadurch, daß der Antriebsmotor ein Hydraulikantriebsaggregat ist, der über ein hydraulisch angetriebenes Kolben-Zylinder-Antriebssystem derart mit der Haltevorrichtung verbunden ist, daß diese in Abhängigkeit der Steuerung des Kolben-Zylinder-Antriebssystems um ihre Achse - schwenkbar ist.

Der Vorteil des hydraulischen Antriebs liegt im wesentlichen darin, daß die abgegebene Leistung gegenüber der bekannten Vorrichtung mit Schrittmotor um das vierbis fünffache vergrößert wird, so daß höhere Winkelgeschwindigkeiten, höhere Drehmomente und höhere Beschleunigungen der Haltevorrichtung erreichbar sind. Darüber hinaus weist die Vorrichtung vorteilhafterweise gegenüber der bekannten Vorrichtung ein sehr viel ruhigeres und gleichmäßigeres Laufverhalten auf, wobei das von der Vorrichtung insgesamt erzeugte Geräuschniveau gegenüber der Vorrichtung mit Schrittmotor erheblich vermindert ist. Auch ist die mechanische Konstruktion der Vorrichtung gegenüber der bekannten Vorrichtung erheblich einfacher im Aufbau und damit kostengünstiger herstellbar, da einerseits eine direkte Ankoppelung des Kolben-Zylinder-Antriebssystems an die Haltevorrichtung möglich ist und andererseits komplizierte Steuerschaltungen und Getriebe sowie eine gesonderte Kupplung, die insgesamt wiederum Fehlerquellen darstellen, vollkommen entfallen. Bedingt durch den Wegfall insbesondere der elektromechanischen Komponenten wird der Betriebsablauf der Vorrichtung insgesamt weniger gestört, so daß beispielsweise Messungen mit der Vorrichtung mit einer wesentlich höheren Auflösung möglich sind als bisher.

Vorteilhafterweise ist im Steuerkreis der Vorrichtung zwischen Kolben-Zylinder-Antriebssystem und Hydraulikantriebsaggregat ein Proportionalventil zur Festlegung vor bestimmter Stellungen der Haltevorrichtungen angeordnet, wobei das Proportionalventil von außen derart steuerbar ist, daß u. a. dessen Ventilstößel beispielsweise in einer Nullstellung und in einer maximalen Vorwärts-und Rückwärtsstellung positioniert werden kann. Das Kolbenzylindersystem ist somit vorwärts und rückwärts über das Proportionalventil steuerbar und umschaltbar, wobei es, unabhängig von der Anzahl der anzutreibenden Haltevorrichtungen in der Gesamtvorrichtung vorzugsweise nur einmal vorkommt.

Da über das Proportionalventil in der Regel immer eine geringe Menge Hydraulikmittel fließt, was zu geringen bzw. langsamen Bewegungen der Haltevorrichtung führen könnte, ist vorzugsweise in der Hydraulikmittelzufuhr und/oder Abfuhrleitung des Steuerkreises zwischen Kolben-Zylinder-Antriebssystem und Hydraulikantriebsaggregat ein Sperrventil der leckfreien Art angeordnet, mit dem vorteilhafterweise sichergestellt wird, daß die Haltevorrichtung, falls gewünscht, absolut bewegungslos gehalten werden kann. Obwohl es nicht zwingend erforderlich ist, kann es zur Erhöhung der Sicherheit vorteilhaft sein, sowohl in die Zufuhrleitung als auch in die Abfuhrleitung des Hydraulikmittels ein derartiges Sperrventil, die beide von außen steuerbar sind, einzufugen.

Vorteilhafterweise sind im Steuerkreis der Vorrichtung zwischen dem Hydraulikantriebsaggregat und Kolben-Zylinder-Antriebssystem und parallel zu diesen zwei Bypass-Ventile angeordnet, wobei das eine Bypass-Ventil einen einstellbaren und das andere einen festen maximalen Hydraulikmitteldruchtritt aufweist. Vorzugsweise durch das Vorsehen dieser Ventile wird es möglich, einerseits ein langsames Absinken der Haltevorrichtung zu erreichen, wenn beispielsweise in einer Notsituation oder bei einer Betriebsstörung der Vorrichtung der Antrieb der Haltevorrichtung unterbrochen werden soll und die Muskulatur eines Patienten durch abruptes Lösen der Haltevorrichtung vom Antrieb ein Verreißen und Überspannen der Muskulatur zur Folge hätte, und andererseits ein schnelles Entkoppeln des Kolben-Zylinder-Antriebssystems vom Antriebsaggregat, wenn eine Extremität in die Haltevorrichtung neu eingespannt werden soll und diesen Vorgang zu erleichtern und zum Durchführen eines Bewegungsablaufes der Haltevorrichtung durch die Bedienungsperson zum Speichern der dabei erfaßten Bewegungsparameter mit der Haltevorrichtung.

Vorteilhafterweise sind das Proportionalventil, die Sperrventile und die Bypass-Ventile jeweils für eine Haltevorrichtung durch einen Rechner steuerbar, so daß der gesamte Betrieb der Haltevorrichtung wiederum sowohl für diagnostische als auch therapeutische Zwecke prozeßgesteuert werden kann. Neben der Steuerung für diese Zwecke erfolgt die Steuerung der Ventile vorzugsweise auch derart, daß bei einem als fehlerhaft erkannten Betriebszustand der Haltevorrichtung der Stromkreis der Ventile und des Hydraulikantriebsaggregats unterbrochen wird, so daß die Ventile ihre Grundstellungen einnehmen. Auf diese Weise wird verhindert, daß die Haltevorrichtung irreguläre Zustände in bezug auf Geschwindigkeit und Stellung einnimmt, so daß Schädigungen von Muskulator und Gelenken der Patienten selbst bei fehlerhaften Betriebszuständen der Vorrichtung ausgeschlossen sind.

Neben dem Erkennen fehlerhafter Betriebszustände durch den die Vorrichtung steuernden Rechner ist es vorteilhaft, daß der Stromkreis der Ventile und des Hydraulikantriebsaggregats nach Auslösung durch eine äußere Schalteinrichtung unterbrochen werden kann, wobei diese äußere Schalteinrichtung ein Notaus-Schalter sein kann, der sowohl vom medizinischen Fachpersonal bzw. Therapeuten als auch vom Patienten ausgelöst werden kann. Auch im Falle der Auslösung durch eine äußere Schalteinrichtung nehmen die Ventile ihre Grundstellungen an.

Gemäß einer vorteilhaften Ausführungsform der Vorrichtung ist in der Grundstellung das Sperrventil bzw. sind die Sperrventile geschlossen, wobei das eine einstellbare Bypass-Ventil geöffnet ist, das andere Bypass-Ventil geschlossen ist und das Hydraulikantriebsaggregat vom Kolben-Zylinder-Antriebssystem abgekoppelt ist, d. h., die Sperrventile sind geschlossen. Das einstellbare Bypass-Ventil ist dabei vorzugsweise so eingestellt, daß in einem derartigen Betriebszustand die Haltevorrichtung langsam absinkt, also ein gedämpftes Absinken der Haltevorrichtung einer stoßgedämpften Absinkbewegung ähnlich vonstatten geht.

Zur Vergößerung der Betriebssicherheit der Vorrichtung insgesamt ist es von Vorteil, daß der Rechner gemäß einer anderen Ausführungsform der Vorrichtung den Schaltzustand der Schalteinrichtung, die in Form eines Notaus-Schalters ausgebildet sein kann, zyklisch überwacht und bei erkannter Schalterauslösung die Ventile steuerseitig inaktiviert. Durch diese zusätzliche Überwachung wird die Betriebssicherheit der Vorrichtung insgesamt noch einmal wesentlich erhöht.

Grundsätzlich möglich ist, für jede Haltevorrichtung ein Hydraulikantriebsaggregat vorzusehen, beispielsweise für drei Haltevorrichtungen in einer Gesamtvorrichtung ist es vorteilhafterweise möglich, daß lediglich ein Hydraulikantriebsaggregat eine Mehrzahl verschiedener Halteeinrichtungen jeweils zugeordneten Kolben-Zylinder-Antriebssystemen antreibt. Aufgrund des erheblichen Leistungsvermögens der Hydraulikantriebsaggregate, die ohne weiteres in der Lage sind, eine Mehrzahl von verschiedenen Kolben-Zylinder-Antriebssystemen anzutreiben, braucht lediglich der jeweiligen Haltevorrichtung ein Satz von Steuerventilen zugeordnet werden, was insgesamt vorteilhafterweise den erforderlichen Steueraufwand gegenüber einer Schrittmotorsteuerung wiederum erheblich vermindert, da dort jeder Haltevorrichtung ein gesonderter Schrittmotor zugeordnet werden muß.

Gemäß einer vorteilhaften Ausführungsform der Vorrichtung ist auf der Achse der Haltevorrichtung eine um die Achse schwenkbare Trägereinrichtung in Form eines im wesentlichen sich in Richtung des freien Endes der Haltevorrichtung erstreckenden Auslegers angeordnet, wobei zwischen dem freien Ende der Haltevorrichtung und dem freien Ende der Trägereinrichtung eine Einrichtung zur Aufnahme des Drehmoments angeordnet ist. Durch diese Art der Anbringung der Einrichtung zur Aufnahme des Drehmoments geht die Masse des Antriebsarms bei der Messung des Drehmoments nicht mit ein, was gegenüber dem Aufbau der bekannten Haltevorrichtung den Vorteil hat, daß die Drehmomente wegen der nicht mehr zu berücksichtigenden Masse der Haltevorrichtung mit größerer Genauigkeit gemessen und kleine Störfehler bei der Geschwindigkeitsmessung einen weitaus geringeren Einfluß auf das Meßergebnis haben, als es bisher der Fall war. Ein zwischen der Haltevorrichtung und der Trägervorrichtung ausgeübtes Drehmoment wird über die Einrichtung zur Aufnahme des Drehmoments erfaßt, wobei vorzugsweise die Einrichtung zur Aufnahme des Drehmoments in Form eines Dehnungsmeßstreifens ausgebildet ist.

Gemäß einer anderen vorteilhaften Ausführungsform ist die Achse der Haltevorrichtung über eine Übersetzungseinrichtung mit einer Meßwinkel-Aufnehmereinrichtung verbunden, wobei diese Übersetzungseinrichtung vorzugsweise in Form einer Seilzugübersetzung ausgebildet ist. Ist die Meßwinkel-Aufnehmereinrichtung vorteilhafterweise in Form eines Winkeldecoders ausgebildet, so wird über die Seilzugübersetzung die Auflösung der Bewegung der Haltevorrichtung derart vergrößert, daß für eine Regelung mit der Bewegung auch bei geringen Drehbewegungen in der Haltevorrichtung ausreichende Informationen erhalten werden, wobei der Seilzug den Vorteil hat, daß damit einerseits ein starrer Antrieb möglich ist und andererseits durch das Vorspannen des Seils die Resonanzfrequenz dieses Systems erheblich über der Regelfrequenz liegt und somit die Regelung nicht negativ beeinflussen kann.

Soll der Arm eines Menschen untersucht bzw. behandelt werden, ist die Vorrichtung vorteilhafterweise so ausgebildet, daß die Achse der der Aufnahme des menschlichen Arms dienenenden Haltevorrichtung gegenüber der im wesentlichen parallel zu der gestellten Längskante der Vorrichtung verlaufenden Auflagelängskante der im wesentlichen rechteckigen Auflageebene des Gestells und parallel zu dieser um einen Winkel gedreht, derart, daß eine gedachte Längsmittellinie durch die Haltevorrichtung einen Winkel zur diesseitigen Auflagelängskante einnimt. Vorteilhafterweise beträgt der Winkel der Achse zur Gestellängskante bzw. zur Auflagelängskante 60°, er kann jedoch auch auf andere Werte einstellbar sein. Aus bewegungsphysiologischen Gründen ist jedoch ein Winkel von 60° besonders vorteilhaft, wobei die Körperlängsachse eines Menschen bei der Aufnahme in der Vorrichtung in Gestellängsrichtung und im wesentlichen parallel zur Gestellängskante bzw. Auflagelängskante verläuft.

Schließlich ist es von Vorteil, daß die Achse der Haltevorrichtung, insbesondere wenn. diese zur Untersuchung und Behandlung eines Arms verwendet wird, gegenüber der Auflageebene des Gestells, die im wesentlichen parallel zu der durch die oberen Gestellträger-Elemente aufgespannten Gestellebene verläuft, in einen Winkel geneigt ist, wobei vorzugsweise dieser Winkel 20° beträgt. Durch diese 20°-Neigung ist es möglich, daß die menschliche Hand bei Bewegung des Armes in der Haltevorrichtung zur Brustmitte des menschlichen Körpers hin bewegt werden kann, was vielfach für diagnostische und therapeutische Zwecke erforderlich ist.

Vorteilhafterweise ist auch dieser Neigungswinkel der Achse nicht starr auf diesen Wert begrenzt, vielmehr ist er auch für andere Anwendungsfälle auf andere Winkel einstellbar, beispielsweise per Hand oder durch steuerbare Einstellmittel, die ebenfalls durch den die Vorrichtung steuernden Rechner gesteuert eingestellt werden können.

Die Erfindung wird nun unter Bezugnahme auf die schematischen Zeichnungen anhand eines Ausführungsbeispieles eingehend beschrieben. Darin zeigen:

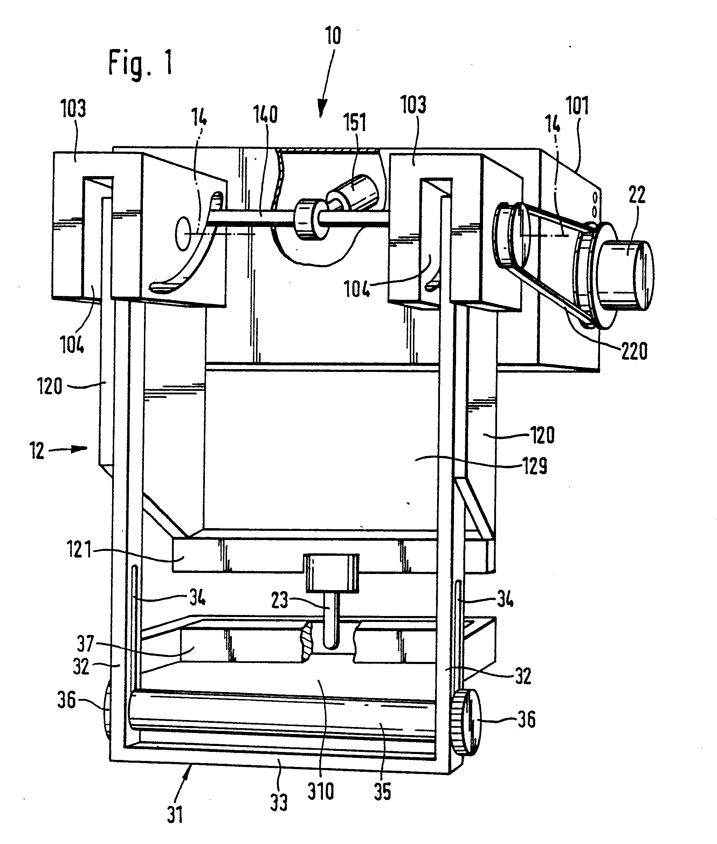

Fig. 1 in perspektivischer Darstellung eine Vorrichtung zur Aufnahme einer Extremität in Form eines menschlichen Armes oder Beines,

Fig. 2 in perspektivischer Darstellung eine Draufsicht auf ein Gestell mit zwei Vorrichtungen zur Aufnahme menschlicher Arme und einer Vorrichtung zur Aufnahme eines menschlichen Beines unter Weglassung einer Auflagefläche für den Menschen,

Fig. 3 eine Seitenansicht der in Fig. 1 dargestellten Vorrichtung im Teilschnitt unter teilweiser Weglassung von Teilen,

Fig. 4 die in Fig. 3 dargestellte Vorrichtung in der Ansicht von hinten im Teilschnitt unter teilweiser Weglassung von Teilen,

Fig. 5 eine Draufsicht auf das in Fig. 2 dargestellte Gestell mit umrißartig dargestellten Menschen unterschiedlicher körperlicher Größe mit teilweise in den Haltevorrichtungen angeordneten Extremitäten und

Fig. 6 ein Blockschaltbild der Vorrichtung zur Darstellung der elektrischen und hydraulischen Steuerungsfunktionszusammenhänge.

Die Vorrichtung 10 besteht im wesentlichen aus einer Haltevorrichtung 12, die der Aufnahme einer Extremität 13 dient und aus einem Antriebsmotor 15 in Form eines Hydraulikantriebsaggregats 150, sowie einem Kolben-Zylinder-Antriebssystem 151, mit dem die Haltevorrichtung 12 geschwenkt wird. Die Haltevorrichtung weist eine geeignet ausgebildete Achse 14 auf, um die diese schwenkbar ist. Die Haltevorrichtung 12 umfaßt zwei im wesentlichen parallel zueinander angeordnete Stege 120, die über einen Quersteg 121 am freien Ende 129 miteinander verbunden sind.

Im wesentlichen parallel zu den Stegen 120 ist eine Trägereinrichtung 31 ausgebildet, die im wesentlichen aus zwei Stegen 32 und einem Quersteg 33 besteht, der am freien Ende 310 der Trägereinrichtung 31 angeordnet ist. Das dem freien Ende 310 gegenüberliegende feste Ende der Stege 32 ist ebenso wie die Stege der eigentlichen Haltevorrichtung 12 und deren Achse 14 drehbar. Die freien Enden der Stege 32 weisen Schlitze 34 auf, in denen ein Handgriff 35 verschiebbar und durch Feststellmittel 36 feststellbar angeordnet ist. Die freien Enden der Stege 32 sind mit einem u-förmigen weiteren Quersteg 37 verbunden, wobei die Schenkel des u-förmigen Querstegs 37 im wesentlichen vertikal nach unten von den Stegen 32 wegweisen. Insgesamt wird durch die Stege 32, den Quersteg 33 und den u-förmigen Quersteg 37 eine muldenförmige Trägereinrichtung 31 gebildet. Zwischen dem freien Ende 129 der Haltevorrichtung 12, das in diesem Falle durch den Quersteg 121 gebildet wird, und dem freien Ende der Trägereinrichtung 310, das in diesem Falle durch den u-förmigen Quersteg 37 gebildet ist eine Einrichtung 23 zur Aufnahme des Drehmoments angebracht. Bei dieser Ausführungsform der Vorrichtung 10 eignet sich als Einrichtung 23 zur Aufnahme des Drehmoments insbesondere ein Dehnungsmeßstreifen.

Die Stege 120 sind in einem Bereich unterhalb der Achse 14 mit einer Verbindungsstange 140 im wesentlichen parallel zur Achse 14 miteinander verbunden. An die Verbindungsstange 140 greift das bewegliche Ende eines Kolben-Zylinder-Antriebssystems 150 an, während das feste Ende des Kolben-Zylinder-Antriebssysteme 150 im unteren Teil eines Vorrichtungsgehäuses in Kolben-Zylinder-Längsrichtung fest aber um eine Achse 102 im wesentlichen parallel zur Achse 14 drehbar angeordnet ist. Die andere Drehachse des Kolben-Zylinder-Antriebssystems 151 ist auf der Verbindungsstange 140 gelagert. Die Achse 14 der Haltevorrichtung 12 ist in zwei geeignet ausgebildeten Lagerböcken drehbar aufgenommen, die auf dem Vorrichtungsgehäuse 101 angebracht sind. In geeignet in den Lagerböcken 103 ausgebildeten Schlitzen 104 sind die Stege 120 und 32 der Haltevorrichtung und der Trägereinrichtung um die Achse 14 drehbar aufgenommen. Ein sich in radial gleichem Abstand von Mittelpunkt der Achse 14 sich erstreckender, in den Lagerböcken 103 ausgebildeter bogenförmiger Schlitz 105 ermöglicht ein Verschwenken der Haltevorrichtung 12 und damit der Trägereinrichtung 31 entsprechend der Auslenkung des Kolben-Zylinder-Antriebssystems 151 um die Achse 14.

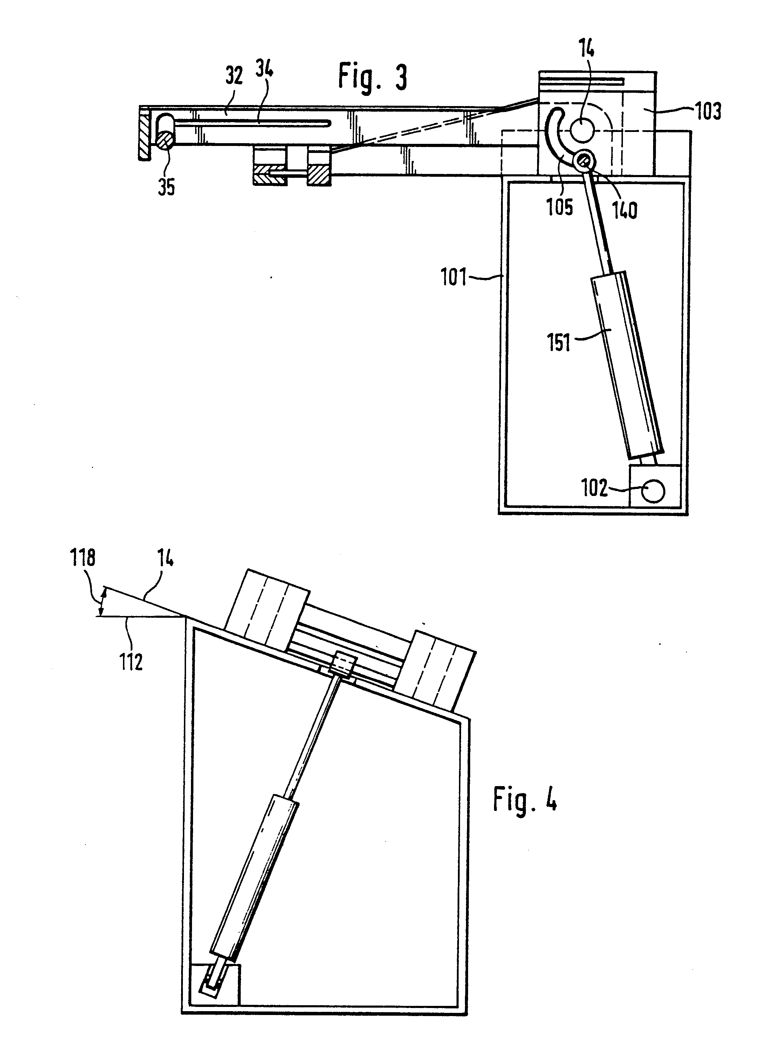

Die Achse 14 ist, wie in Fig. 4 dargestellt, um einem Winkel von ungefähr 20° gegenüber der Horizontalen, die durch eine Ruhefläche bzw. später noch beschriebene Auflageebene 112 dargestellt wird, geneigt.

Die Achse 14 ist über eine Übersetzungseinrichtung 220 mit einer Meßwinkel-Aufnehmereinrichtung 22 verbunden, wobei die Übersetzungseinrichtung in Form einer Seilzugübersetzung ausgebildet ist. Diese Meßwinkelaufnehmereinrichtung 22 dient der Erfassung der Stellung des menschlichen Arms von 0 - 120° bzw. der Stellung der Haltevorrichtung 12 gedreht um die Achse 14, wobei die Meßwinkelaufnehmer-Einrichtung hier in Form eines Winkeldecoders ausgebildet ist. Die Auflösung dieses Winkeldecoders beträgt 5.000 Löcher bei 360° Bewegung. Mittels elektronischer Vervierfachung wird hier eine Auflösung bei einer Bewegung von 120° von 4 x 5.000 dividiert durch 3 erreicht. Diese Auflösung ist zur Bestimmung einer bestimmten Position voll ausreichend, für die Regelung ist jedoch eine höhere Auflösung nötig, da die Regelung mit einem Zeitraster von 4 msec. erfolgt und innerhalb von 4 msec. ein nennenswerter Weg zurückgelegt werden muß, damit Informationen erzeugt werden, mit denen eine Regelung bzw. Steuerung erfolgen kann. Aus diesem Grund wird die Auflösung mit der Übersetzungs-Einrichtung 220 in Form einer Seilzugübersetzung um den Faktor 5,6 erhöht. Die Auflösung beträgt dann 4 x 5.000 : 3 x 5,6. Diese Auflösung ist ausreichend, um für die Regelung bzw. Steuerung benötigte Informationen zu erhalten. Ein Seilzug als Übersetzungs-Einrichtung 220 hat den Vorteil, daß damit einerseits ein starrer Antrieb möglich ist und zum anderen durch ein Vorspannen des Seils mit einer Kraft von ca. 100 N die Resonanzfrequenz eines derartigen Übertragungssystems bei etwa 300 Hz liegt. Diese hohe Resonanzfrequenz ist deshalb erforderlich, da die Regelfrequenz der Vorrichtung 10 bei 50 -100 Hz liegt und die Resonanzfrequenz dieses Systems die Regelung somit nicht beeinflussen kann.

Ein Vorrichtung 10 umfaßt, wie durch die gestrichelte Linie dargestellt, neben der Haltevorrichtung 12 und dem diese unmittelbar antreibenden Kolben-Zylinder-Antreibssystem 151 jeweils ein in der Hydraulikmittel-Zufuhrleitung 154 und in der Hydraulikmittel-Abfuhrleitung 155 angeordnetes Sperrventil 156, 157 sowie jeweils dem Kolben-Zylinder-Antriebssystem 151 in den Hydraulikmittel-Zufuhr-und -Abfuhrleitungen 154, 155 angeordnete Bypass-Ventile 158, 159. Das eine Bypass-Ventil 158 weist einen einstellbaren und das andere 159 einen festen maximalen Hydraulikmittel-Durchtritt auf.

In den Hydrauliksteuerkreis 153 zwischen Kolben-Zylinder-Antriebs-System 151 und Hydraulikantriebsaggregat 150 ist ein Proportionalventil 152 zur Festlegung vorbestimmter Stellungen der Haltevorrichtung 12 angeordnet. Das Proportionalventil 152 wird von einer Ansteuerungselektronikeinrichtung 27 gesteuert, die mit einem Rechner 24 verbunden ist. Das Proportionalventil 152 hat eine eigene Lageregelung, was bedeutet, daß der Ventilstößel in einem eigenen Regelkreis auf einer Stellung gehalten werden kann, wie sie von außen vorgegeben werden kann, d. h., die Stellung dieses Stößels vom Rechner 24 vorgegeben. Der Stößel kann dadurch in einer Null-Stellung, in einer maximalen Vorwärts-und in einer maximalen Rückwärtsstellung positioniert werden. Somit ist das Kolben-Zylinder-Antriebssystem 151 über das Proportionalventil steuerbar.

Der Rechner 24 steuert und überwacht ebenfalls die im Steuerkreis 153 angeordneten Sperrventile 156, 157, und die Bypass-Ventile 158, 159 und überwacht zyklisch deren Betriebsverhalten sowie das Betriebsverhalten des Proportionalventils 152. Zum Rechner 24 gehen ebenfalls von der Meßwinkel-Aufnahmeeinrichtung 22 sowie die Einrichtung zur Aufnahme des Drehmoments 23, wobei die Signale von der Einrichtung 23 zur Aufnahme des Drehmoments wiederum über einen Verstärker 231, einen Tiefpaßfilter 232, einen Verstärker 233 und über einen Analog-Digitalwandler 234 zum Rechner 24 geführt werden.

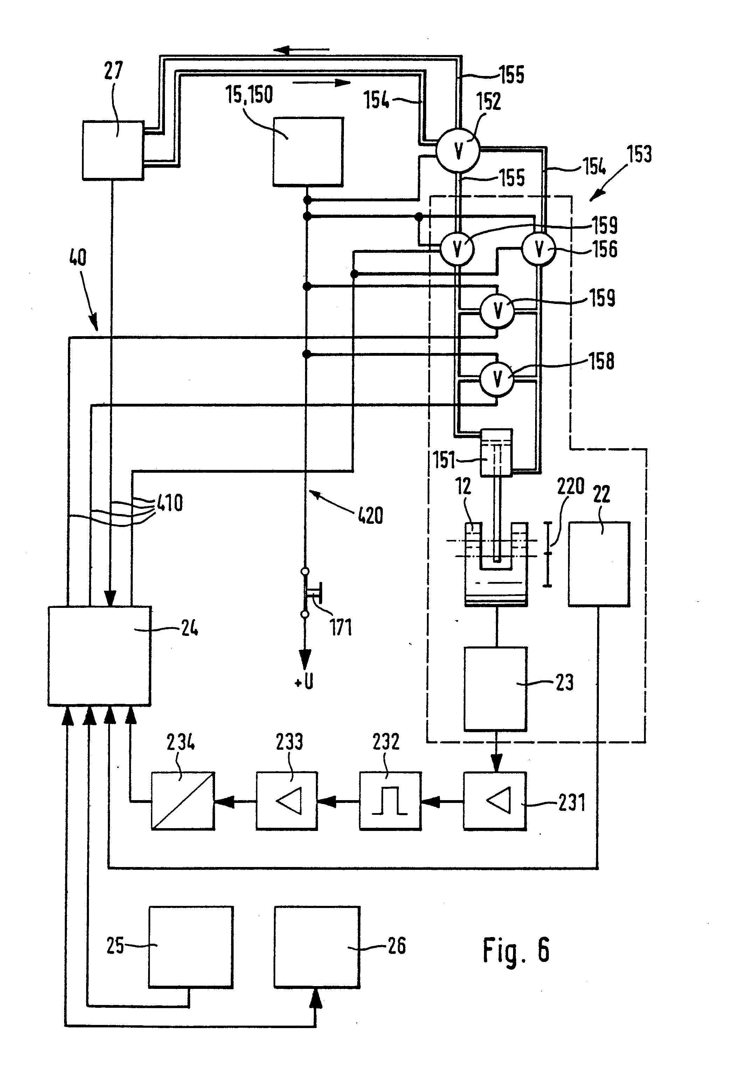

Die Ventile 152, 156, 157, 158 und 159 sowie das Hydraulikantriebsaggregat 150 weisen neben dem hydraulischen Steuerkreis 153 einen elektrischen Steuerkreis 40 auf, der sich aus Steuerkreis 410 zur elektronischen Regelung und Überwachung der Ventile und des Hydraulikantriebsaggregat und aus dem Stromversorgungskreis 420 zusammensetzt, der die Ventile und das Hydraulikantriebsaggregat zu deren Antrieb versorgt. In den Stromversorgungskreis 420 ist die äußere Schalteinrichtung 171, die in Fig. 6 symbolisch als Austaster angedeutet ist, eingefügt. Die Schalteinrichtung 171 unterbricht den Stromversorgungskreis der Ventile und des Hydraulikantriebsaggregats 159 dann, wenn eine Bedienungsperson ihn betätigt, beispielsweise bei fehlerhaft erkannten Betriebszuständen insgesamt oder dann, wenn der Patient sie bei subjektiver oder objektiver Gefahr betätigt. Bei einem als fehlerhaft erkannten Betriebszustand, bei dem der Stromversorgungskreis 420 der Ventile und des Hydraulikantriebsaggregats 150 unterbrochen wird, nehmen die Ventile ihre Grundstellungen ein, d. h., die Sperrventile 156, 157 sind geschlossen, während das einstellbare Bypass-Ventil 158 geöffnet ist und das andere Bypass-Ventil 159 geschlossen ist. Das Hydraulikantriebsaggregat 150 wird ebenfalls vom Kolben-Zylinder-Antriebssystem 150 abgekoppelt.

Da der Rechner 24 den Schaltzustand der Schalteinrichtung 171 neben den Ventilen und den übrigen Steuereinrichtungen der Vorrichtung 10 zyklisch überwacht, werden bei erkannter Auslösung der Schaltereinrichtung 171 die Ventile zusätzlich über die Steuerkreise 410 inaktiviert. Dadurch wird infolge der zusätzlichen Überwachung der Schalteinrichtung 171 durch den Rechner 24 wesentlich erhöht, so daß fehlerhafte Betriebszustände der Vorrichtung 10 niemals zu einer Schädigung des Menschen führen können, da die Vorrichtung 10 dann sofort ausgeschaltet wird. Bei einem derartigen Betriebszustand, unabhängig davon, ob der Rechner 24 oder die Schaltereinrichtung 171 die Ventile ausgeschaltet hat, ermöglicht dann das in seine Grundstellung übergegangene einstellbare Bypass-Ventil 158, das in dieser Stellung geöffnet ist, ein langsames Absinken der Haltevorrichtung 12, so daß die menschliche Muskulatur sich langsam entspannen bzw. das menschliche Gelenk langsam in eine Ruheposition überführt werden kann, ohne daß ein Verreißen oder Überspannen der Muskulatur bzw. der Gelenke befürchtet werden muß.

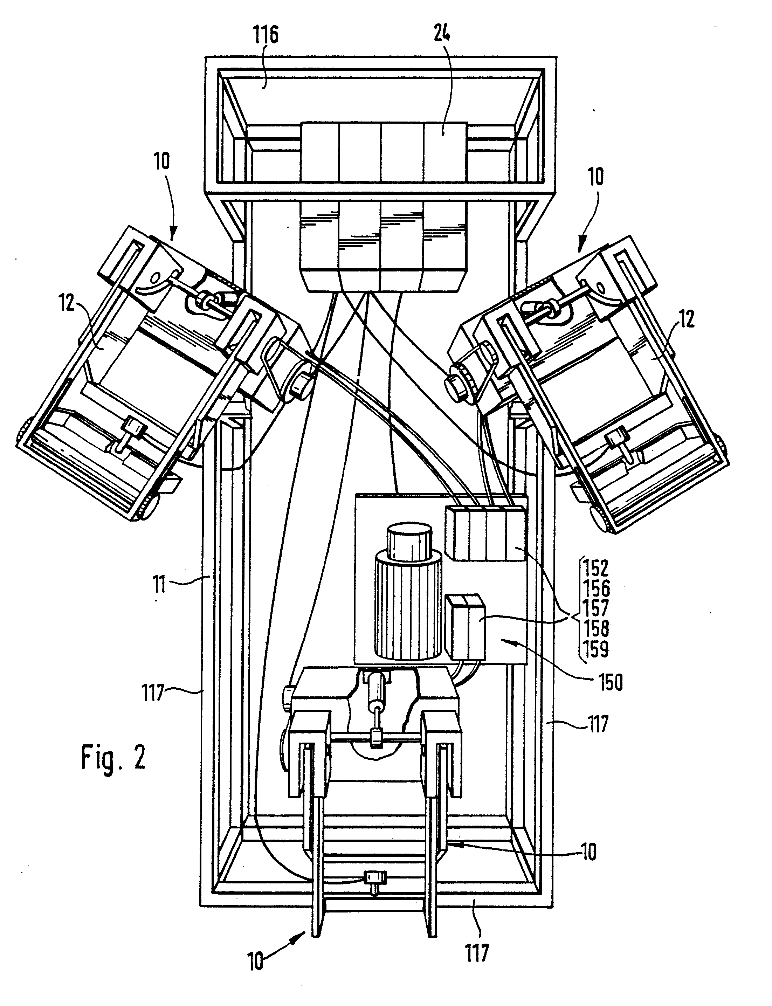

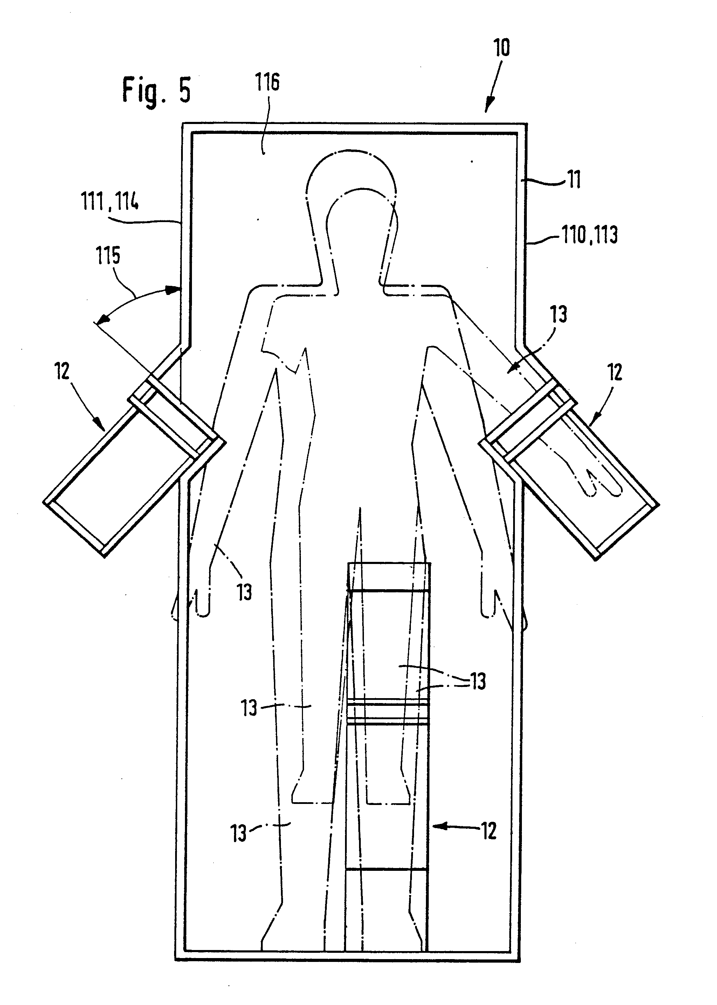

Eine Vorrichtung der beschriebenen Art kann auch in einer Mehrzahl in einem Gestell 11 untergebracht sein und gemeinsam betrieben werden, wie es schematisch in den Fig. 2 und 5 dargestellt ist. Das Gestell 11 nimmt gem. der Darstellung drei Haltevorrichtungen 12 auf, wobei zwei für jeweils einen Arm des menschlichen Körpers und eine für ein Bein eines menschlichen Körpers vorgesehen sind. Es sind jedoch auch beliebige andere Kombinationen möglich. Anders als für die Haltevorrichtung 12, die für ein menschliches Bein benutzt wird, sind die Achsen 14 der Haltevorrichtungen 12, die für die menschlichen Arme benutzt werden, gegenüber der im wesentlichen parallel zu der Gestellängskante 110, 111 verlaufenden Auflagelängskante 113, 114 der im wesentlichen rechteckigen Auflageebene 112 des Gestells 11 und parallel zu dieser um einen Winkel 115 gedreht. Dieser Winkel 115 beträgt ca. 60°, wobei sich dieser Winkel als für die Diagnose als auch für die Therapie des menschlichen Arms als äußerst günstig herausgestellt hat.

Wie auch aus Fig. 4 ersichtlich ist, ist die Achse 14 der Haltevorrichtung, wenn sie für einen menschlichen Arm benutzt wird, gegenüber der Auflageebene 112 des Gestells, die im wesentlichen parallel zu der durch die oberen Gestellträgerelemente 117 aufgespannten Gestellebene 116 verläuft, in einem Winkel 118 geneigt, wobei der Winkel 118 vorteilhafterweise 20° beträgt, da dann eine Führung der Hand des eingespannten Armes zur Brustmitte hin ermöglicht wird. Beide Winkel 115 mit 60° und 118 mit 20° ergeben unter medizinischen Gesichtspunkten eine optimale und einwandfreie Bewegung des Armes.

Durch geringfügige Veränderung der Haltevorrichtung 12 und ggfs. auch der Winkel 115 und 118 ist eine Diagnose und Therapie des Hüftgelenkes oder auch der Schulter eines Menschen möglich, wobei dann statt der Unterschenkel der Oberschenkel und beim Arm statt des Unterarmes der Oberarm auf der Haltevorrichtung 12 befestigt ist. Aus Gründen der Übersichtlichkeit ist eine Einrichtung zum Festhalten der Extremität in der Haltevorrichtung 12 hier nicht dargestellt, es handelt sich jedoch dabei um Befestigungsmittel, mit denen die menschliche Extremität zwar starr aber schmerzfrei in der Haltevorrichtung 12 befestigt werden kann.

Nach dem Betten einer Person 13 auf der Auflageebene 112 des Gestells 11 wird eine oder werden mehrere Extremitäten in die jeweilige Haltevorrichtung 12 der Vorrichtung bzw. Vorrichtungen 10 eingelegt und dort mittels der hier nicht dargestellten Einrichtung zum Festhalten der Extremität fixiert.

Von der Bedienungsperson werden nun über eine dem Rechner 24 zugeordnete Eingabetastatur neben dem Datum, der Uhrzeit, der Bezeichnung der Person und der Bedienungsperson und einem evtl. Kommentar, die bewegungsspezifischen Parameter wie Bewegungsdauer, Geschwindigkeit, Beschleunigung, Winkelbereich und maximal einzuhaltendes Drehmoment sowie Angaben zur Formatierung der ermittelten Werte bei der Darstellung in an den Rechner angeschlossenen Aufzeichnungsmitteln 25 bzw. Anzeigemitteln 26 eingegeben. Vor der eigentlichen Aufnahme der Extremität 13 in der Vorrichtung erfolgt jedoch nach jedem Neueinschalten des Geräts, beispielsweise 1x am Tag, die Aufnahme einer Eichkurve der Vorrichtung, d. h., es werden die Reibung in den Lagern der Haltevorrichtung 12, die durch das Gewicht der Haltevorrichtung 12 und die durch seine Trägheit verursachten Meßfehler und andere Meßfehler verursachende Parameter mit der leeren Haltevorrichtung 12 ermittelt, so daß diese nachfolgend bei der Darstellung der für die Bewegung der Haltevorrichtung 12 mit eingelegter Extremität 13 typischen Bewegungsparameter berücksichtigt werden können, indem sie minimalisiert werden.

Nach Eingabe der Parameter in Rechner 24 werden die Ventile auf geeignete Weise aktiviert und die Haltevorrichtung 12 wird beispielsweise auf "Meßbereich Anfang Streckung" positioniert. Nachfolgend erfolgt die Messung "Streckung". Daran anschließen kann beispielsweise ein Positionieren auf "Meßbereich Anfang Beugung" erfolgen und nachfolgend die Messung "Beugung". Anschließend werden die Ventile geeignet deaktiviert. Nachfolgend erscheint beispeilsweise der oder die ermittelten Meßwerte, beispielsweise in Form eines auf dem Aufzeichnungsmittel 25 dargestellten Diagramms. Dieser für den Diagnostikfall typische Verfahrensablauf kann anschließend beliebig oft unter Variation einzelner Bewegungsparameter wiederholt werden und erlaubt eine objektive und quantitative beliebig oft reproduzierbare Erfassung des Grades des Grades der Funktionsfähigkeit des jeweils untersuchten Bewegungsapparates eines Menschen.

Für therapeutische Zwecke vollzieht sich die Eingabe der Parameter in den Rechner 24 auf gleiche Weise und nachdem diese abgeschlossen ist, vollführt die Vorrichtung mit in der Haltevorrichtung 12 eingelegter Extremität die den angegebenen Befehlen entsprechenden Bewegungsabläufe. Zu diesem Zweck ist es möglich, daß der Bewegungsablauf für therapeutische oder Trainingszwecke anhand eines bestimmten vorgegebenen Programms durchgeführt wird, es ist jedoch auch möglich, daß die Bedienungsperson, die von der Vorrichtung 10 nachfolgend automatisch die zu durchlaufenden Bewegungszyklen zunächst durch Bewegung der Haltevorrichtung 12 in Echtzeit vorgibt und der Rechner 24 dabei die durch die Bedienungsperson geführte Bewegung der Haltevorrichtung in bezug auf Beschleunigung und Geschwindigkeit analysiert und speichert, so daß die Vorrichtung 10 anhand dieser Werte die Bewegung bei der Therapie selbständig oft ausführen kann.

Schließlich besteht noch die Möglichkeit einer Verknüpfung von Therapie und Diagnose, indem beispielsweise zunächst die Parameter einer Person für diagnostische Zwecke gemessen werden, nach folgender dann eine bestimmte Anzahl von Therapiebewegungen durchgeführt wird und dann anschließend wiederum eine Messung der Person zu diagnostischen Zwecken erfolgt und schließlich nachfolgend die Auswertung durch den Rechner 24. Darüber hinaus sind beliebige andere Kombinationen von Therapie und Diagnoseschritten .mit dem hier beschriebenen Verfahren unter Vorrichtung möglich, je nach der Art der Funktionsstörung des Bewegungsapparates des Menschen. Sowohl beim Einsatz der Vorrichtung 10 für diagnostische Zwecke als auch zum Zwecke der Therapie wird vom Rechner ständig das von der Bedienungsperson eingestellte maximale Drehmoment der Haltevorrichtung, seine Winkelstellung, das Auslösen einer Notsituation durch Drücken der äußeren Schalteinrichtung 171 und das Auslösen einer Notsituation durch Eingabe beliebiger Tasten am Terminal überwacht.

Vom Rechner 24 wird nach der Ermittlung der entsprechenden Bewegungsparameter das maximal vorkommende Drehmoment für Beugung und Streckung, die Drehmomentarbeit für Beugung und Streckung, die Hysterese (Arbeit Beugung-Arbeit Streckung) und die Leistung für Beugung und Streckung berechnet. Diese Werte werden vorzugsweise im Klartext auf einem Aufzeichnungsmittel 25, beispielsweise einem Plotter dargestellt. Ebenfalls dargestellt werden auf dem Aufzeichnungsmittel 25 in graphischer Form ein Winkel-Zeit-Diagramm für Beugung und Streckung und ein Fourier-Spektrum für Beugung und Streckung. Das dargestellte Fourier-Spektrum gibt eine Aussage über die Frequenzanteile, die bei Winkeländerungen der Haltevorrichtung 12 bei der Messung auftreten, d. h., die vom Verdrehungswinkel abhängigen und vom statistischen bzw. dynamischen Betrieb abhängigen überlagerten Meßwertschwankungen, die relativ zum jeweiligen bzw. momentanen Meßzustand existieren, können vorzugsweise im Sinne einer Fourier-Analyse ausgewertet werden.

BEZUGSZEICHENLISTE

10 Vorrichtung
101 Vorrichtungsgehäuse
102 Achse
103 Lagerbock
104 Schlitz
105 kreisförmier Schlitz
11 Gestell
110 Gestellängskante

111 Gestellängskante
112 Auflageebene
113 Auflagelängskante
114 Auflagelängskante
115 Winkel
116 Gestellebene
117 Gestellträgerelement
118 Winkel
12 Haltevorrichtung
120 Steg
121 Quersteg
129 freies Ende der Haltevorrichtung
13 Mensch
14 Achse
15 Antriebsmotor
150 Hydraulikantriebsaggregat
151 Kolben-Zylinder-Antriebssystem
152 Proportionalventil
153 Steuerkreis
154 Hydraulikmittel-Zufuhrleitung
155 Hydraulikmittel-Abfuhrleitung
156 Sperrventil
157 Sperrventil
158 einstellbares Bypass-Ventil
159 Bypass-Ventil
171 äußere Schalteinrichtung
22 Meßwinkel-Aufnahme-Einrichtung
220 Meßwinkel-Übersetzungs-Einrichtung
23 Einrichtung zur Aufnahme des Drehmoments
24 Rechner
27 Ansteuerungs-Elektronik-Einrichtung
31 Trägereinrichtung
310 freies Ende der Trägereinrichtung
32 Steg
33 Quersteg
34 Schlitze
35 Handgriff
36 Festellmittel
37 u-förmiger Quersteg
40 elektrischer Steuerkreis
410 Steuerkreis für elektonische Regelung und Überwachung
420 Stromversorgungskreis

## Ansprüche

1. Vorrichtung zur Ermittlung und Verbesserung der Funktionsfähigkeit des Bewegungsapparates des Menschen, insbesondere seiner Extremitäten, mit einer der Aufnahme der extremitätedienenden Haltevorrichtung, die durch einen Antriebsmotor um eine in der Haltevorrichtung aufgenommenen Gelenkachse einer Extremität entsprechenden Achse drehbar ist, dadurch gekennzeichnet, daß der Antriebsmotor (15) ein Hydraulikantriebsaggregat (150) ist, der über ein hydraulisch angetriebenes Kolben-Zylinder-Antriebssystem (151) derart mit der Haltevorrichtung (12) verbunden ist, daß diese in Abhängigkeit von der Steuerung des Kolben-Zylinder-Antriebssystems (151) um ihre Achse (14) schwenkbar ist.

2. Vorrichtung nach Anspruch 1, dadurch gekennzeichnet, daß im Steuerkreis (153) zwischen Kolben-Zylinder-Antriebs-System (151) und Hydraulik-Antriebs-Aggregat (150) ein Proportionalventil (152) zur Festlegung vorbestimmter Stellungen der Haltevorrichtung (12) angeordnet ist.

3. Vorrichtung nach einem oder beiden der Ansprüche 1 oder 2, dadurch gekennzeichnet, daß in der Hydraulikmittelzufuhr-und/oder -abfuhrleitung (154, 155) des Steuerkreises (153) zwischen Kolben-Zylinder-Antriebssytem (151) und Hydraulikantriebsaggregat (150) ein Sperrventil (156, 157) der leckölfreien Art angeordnet ist.

4. Vorrichtung nach einem oder mehreren der Ansprüche 1 bis 3, dadurch gekennzeichnet, daß im Steuerkreis (153) zwischen Hydraulikantriebsaggregat (150) und Kolben-Zylinder-Antriebssystem - (151) und parallel zu diesem zwei Bypassventile - (158, 159) angeordnet sind, wobei das eine Bypassventil (158) einen einstellbaren und das andere (159) einen festen maximalen Hydraulikmitteldurchtritt aufweist.

5. Vorrichtung nach einem oder mehreren der Ansprüche 2 bis 4, dadurch gekennzeichnet, daß das Proportionalventil (152) die Sperrventile (156, 157) und die @ypassventile (158, 159) jeweils für eine Haltevorrichtung (12) durch einen Rechner - (24) steuerbar sind.

6. Vorrichtung nach einem oder mehreren der Ansprüche 2 bis 5, dadurch gekennzeichnet, daß die Steuerung der Ventile derart erfolgt, daß bei einem als fehlerhaft erkannten Betriebszustand der Haltevorrichtung (12) der Stromkreis der Ventile und des Hydraulikantriebsaggregats (150) unterbrochen wird, so daß die Ventile ihre Grundstellungen einnehmen.

7. Vorrichtung nach Anspruch 6, dadurch gekennzeichnet, daß der Stromkreis der Ventile und des Hydraulikantriebsaggregats (150) nach Auslösung durch eine äußere Schalt einrichtung - (171) unterbrochen wird.

8. Vorrichtung nach einem oder beiden der Ansprüche 6 oder 7, dadurch gekennzeichnet, daß in der Grundstellung das Sperrventil (156, 157) geschlossen ist, wobei das eine einstellbare Bypassventil (158) geöffnet ist, das andere Bypassventil - (159) geschlossen ist und das Hydraulikantriebsaggregat (150) vom Kolben-Zylinder-Antriebssystem - (150) abgekoppelt ist.

9. Vorrichtung nach Anspruch 7, dadurch gekennzeichnet, der Rechner (24) den Schaltzustand der Schalteinrichtung (171) zyklisch überwacht und bei erkannter Schalterauslösung die Ventile inaktiviert.

10. Vorrichtung nach einem oder mehreren der Ansprüche 1 bis 9, dadurch gekennzeichnet, daß das Hydraulikantriebsaggregat (150) einer Mehrzahl verschiedener Haltevorrichtungen (12) jeweils zugeordneten Kolben-Zylinder-Antriebssystemen - (151) antreibt.

11. Vorrichtung nach einem oder mehreren der Ansprüche 1 bis 10 dadurch gekennzeichnet, daß auf der Achse (14) der Haltevorrichtung (12) eine um die Achse (14) schwenkbare Trägervorrichtung (31) in Form eines im wesentlichen sich in Richtung des freien Endes (129) der Haltevorrichtung - (12) erstreckenden Auslegers angeordnet ist, wobei zwischen dem freien Ende (129) der Haltevorrichtung (12) und dem freien Ende (310) der Trägereinrichtung (31) eine Einrichtung (23) zur Aufnahme des Drehmoments angeordnet ist.

12. Vorrichtung nach Anspruch 11 dadurch gekennzeichnet, daß die Haltevorrichtung (12) über die Trägereinrichtung (31) durch das Kolben-Zylinder-Antriebssystem (151) schwenkbar ist, wobei ein zwischen Haltevorrichtung (12) und Trägereinrichtung (31) ausgeübtes Drehmoment durch die Einrichtung (23) zur Aufnahme des Drehmoments erfaßbar ist.

13. Vorrichtung nach einem oder mehreren der Ansprüche 1 bis 12, dadurch gekennzeichnet, daß die Achse (14) der Haltevorrichtung (12) über eine Übersetzungseinrichtung (220) mit einer Meßwinkelaufnehmereinrichtung (22) verbunden ist.

14. Vorrichtung nach Anspruch 13 dadurch gekennzeichnet, daß die Übersetzungseinrichtung - (200) in Form einer Seilzugübersetzung ausgebildet ist.

15. Vorrichtung nach einem oder mehreren der Ansprüche 1 bis 13, dadurch gekennzeichnet, daß die Achse (14) der Haltevorrichtung (12) gegenüber der im wesentlichen parallel zu der Gestellängskante (110, 111) verlaufenden Auflagelängskante (113, 114) der im wesentlichen rechteckigen Auflageebene (112) des Gestells (11) und parallel zu dieser um einem Winkel (115) gedreht ist.

16. Vorrichtung nach Anspruch 15, dadurch gekennzeichnet, daß der Winkel (115) 60° beträgt.

17. Vorrichtung nach einem oder mehreren der Ansprüche 1 bis 16 dadurch gekennzeichnet, daß die Achse (14) der Haltevorrichtung (12) gegenüber der Auflageebene (112) des Gestells (11), die im wesentlichen parallel zu der der die obere Gestellträgerebene (117) aufgespannten Gestellebene (116) verläuft, in einem Winkel (118) geneigt ist.

18. Vorrichtung nach Anspruch 17, dadurch gekennzeichnet, daß der Winkel (118) 20° beträgt.

Fig. 1

Fig. 2

**Fig. 3**

**Fig. 4**

Fig. 5

Fig. 6

Europäisches
Patentamt

**EUROPÄISCHER RECHERCHENBERICHT**

Nummer der Anmeldung

## EINSCHLÄGIGE DOKUMENTE

EP 85112339.8

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US - H - T 100 602 (ROLEY) | 1 | A 61 B 5/10 |
| A | * Zusammenfassung; Fig. 1,2 * | 11 | A 61 H 1/00 |
| | -- | | A 63 B 21/00 |
| Y | US - A - 3 822 599 (BRENTHAM) | 1 | |
| A | * Zusammenfassung; Spalte 2, Zeilen 17-48; Fig. 3,4 * | 2 | |
| | -- | | |
| A | DE - B2 - 1 478 056 (PERRINE) | 1-4 | |
| | * Spalte 1; Ansprüche 1,4,6,10, 12; Fig. 1-6 * | | |
| | -- | | |
| A | GB - A - 2 152 381 (MOOMAW) | 1,2 | |
| | * Zusammenfassung; Seite 2, Zeilen 32-36, 86-98; Fig. 1, 2 * | | |
| | -- | | RECHERCHIERTE SACHGEBIETE (Int. Cl.4) |
| A | DE - A - 1 578 544 (CUINIER) | 1,2,11 | A 61 B |
| | * Seite 2, Absätze 4,5; Seite 19; Ansprüche 1,3,5; Fig. 5, 13 * | | A 61 H |
| | | | A 63 B |
| | -- | | |
| A | US - A - 3 575 159 (PILE) | 1,13, 14 | |
| | * Zusammenfassung; Spalte 2, Zeile 74 - Spalte 3, Zeile 7; Fig. 8 * | | |
| | ---- | | |

Der vorliegende Recherchenbericht wurde für alle Patentansprüche erstellt.

| Recherchenort | Abschlußdatum der Recherche | Prüfer |
|---|---|---|
| WIEN | 26-03-1986 | NEGWER |